# EUROPÄISCHE PATENTSCHRIFT

(11) **EP 0 902 009 B1**
(45) Veröffentlichungstag und Bekanntmachung des Hinweises auf die Patenterteilung: **27.10.2004**
(21) Anmeldenummer: 98116083.1
(22) Anmeldetag: 26.08.1998
(51) Int. Cl.: C07C 219/10, C07C 217/08, A61K 7/42, A61K 7/50

(54) **Esterquats auf Zimtsäurebasis**
Esterquats based on cinnamic acid
Esterquats à base d'acide cinnamique

(30) Priorität: 04.09.1997 DE 19738641
(43) Veröffentlichungstag der Anmeldung: 17.03.1999
(73) Patentinhaber: Cognis Deutschland GmbH & Co. KG, 40589 Düsseldorf (DE)
(72) Erfinder: Copete Vidal, Teresa Dr., 08037 Barcelona (ES); Ponsati Obiols, Oriol Dr., 08005 Barcelona (ES); Pi Subirana, Rafael Dr., 084000 Granollers (ES); Bigorra Llosas, Joaquin Dr., 08203 Sabadell (ES); Uphues, Günter, 40789 Monheim (DE)

(56) Entgegenhaltungen:
- EP-A- 0 272 576
- EP-A- 0 834 304
- DE-C- 957 162
- FR-A- 2 504 530

## Beschreibung

### Gebiet der Erfindung

Die Erfindung betrifft neue Esterquats auf Zimtsäurebasis sowie deren Verwendung als Lichtschutzfilter in der Kosmetik und Textiltechnik.

### Stand der Technik

Zum Schutz gegen die schädlichen Auswirkungen von UV-Strahlen werden kosmetischen Produkten vielfach Lichtschutzfilter zugegeben, die in der Lage sind, ultraviolette Strahlen zu absorbieren und die aufgenommene Energie in Form längerwelliger Strahlung, z.B. Wärme wieder abzugeben. Typische Beispiele sind Aminobenzoesäureester, Methoxyzimtsäure, Benzophenone, Dibenzoylmethane, Salicylsäureester und dergleichen, aber auch feindisperse Metalloxide, wie z.B. Titandioxid oder Zinkstearat. Diese Stoffe, zumal in synergistischen Kombinationen, erfüllen die Anforderungen an den Lichtschutz zufriedenstellend, das Problem besteht jedoch darin, sie in größeren Mengen stabil zu dispergieren. Ganz konkret besteht im Markt der Wunsch nach Lichtschutzfiltern, die eine hohe UV-Absorption besitzen und gut löslich in polaren Medien sind. Nach Möglichkeit sollten diese Stoffe noch weitere vorteilhafte Eigenschaften besitzen, insbesondere Haut und Haaren einen angenehmen Weichgriff verleihen und die elektrostatische Aufladung herabsetzen. Für derartige Stoffe besteht auch im Bereich der Textiltechnik zur Ausrüstung von Stoffen gegen Ausbleichung ein großer Markt.

Die komplexe Aufgabe der Erfindung hat darin bestanden, neue Lichtschutzfilter sowohl für die Kosmetik als auch für die Textiltechnik zur Verfügung zu stellen, die über oberflächenaktive Eigenschaften verfügen, auf der Haut einen leichten sensorischen Eindruck hinterlassen, sowohl natürlichen wie auch synthetischen Fasern, also Haaren und Textilien, einen angenehmen Weichgriff verleihen und die elektrostatische Aufladung zwischen den Fasern herabsetzen und dabei gleichzeitig noch über UVabsorbierende Eigenschaften verfügen.

### Beschreibung der Erfindung

Gegenstand der Erfindung sind Esterquats, deren Säurekomponente sich ganz oder teilweise von Zimtsäure, ableitet.

Überraschenderweise weisen die genannten Stoffe nicht nur ausgezeichnete oberflächenaktive, avivierende und antistatische Eigenschaften auf, sie besitzen auch ein ausgezeichnetes Absorptionsvermögen für UV-Strahlen und eignen sich daher beispielsweise als UV-Filter für kosmetische Produkte wie Sonnenschutzmittel, Haut- und Haarpflegemittel sowie zur Ausrüstung von Textilien gegen Ausbleichen. Ein weiterer Vorteil der Stoffe liegt in ihrer guten Löslichkeit, was es ermöglicht, die neuen Esterquats in größeren Mengen dauerhaft in Formulierungen einzuarbeiten. Da die Stoffe ihrerseits emulgierende Eigenschaften besitzen, kann auf diese Weise sogar zusätzlich noch die Menge an Emulgator in den Mitteln verringert werden, was aus ökonomischer Sicht natürlich stets gewünscht wird.

### Esterquats

Unter der Bezeichnung Esterquats" werden im allgemeinen quaternierte Triethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Carbonsäuren partiell verestert, Luft durchleitet und anschließend mit Dimethylsulfat oder Ethylenoxid quaterniert. Übersichten zu diesem Thema sind beispielsweise von R.Puchta et al. in **Tens.Surf.Det. 30, 186 (1993),** M.Brock in **Tens.Surf.Det. 30, 394 (1993),** R.Lagerman et al. in **J.Am.Oil.Chem.Soc. 71, 97 (1994)** sowie I.Shapiro in **Cosm.Toil. 109, 77 (1994)** erschienen.

Die quaternierten Zimtsäuretriethanolaminestersalze folgen der Formel **(I)**, 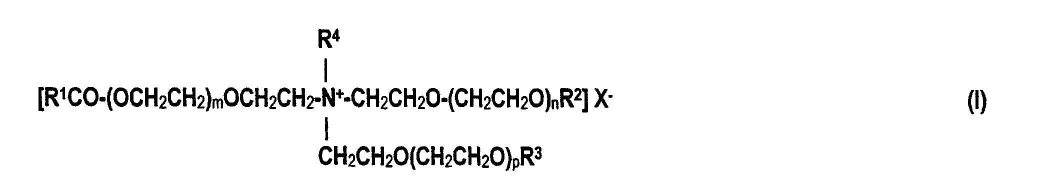 in der R¹CO für einen Rest der Zimtsäure, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

Die erfindungsgemäßen Esterquats können sich vollständig von Zimtsäuren ableiten. Es ist ferner ebenfalls möglich, daß die Zimtsäure nur einen Anteil von 10 bis 90 Mol-% an der Gesamtsumme der Säurekomponente ausmacht. Demzufolge können auch Mischungen von Zimtsäure beispielsweise mit Fettsäuren mit 6 bis 22, vorzugsweise 12 bis 18 Kohlenstoffatomen im Molverhältnis 10 : 90 bis 90 : 10, vorzugsweise 25 : 75 bis 75 : 25 und insbesondere 40 : 60 bis 60 : 40 eingesetzt werden. Weiterhin ist es möglich, Mischungen von Zimtsäure mit Dicarbonsäuren mit 2 bis 12, vorzugsweise 4 bis 6 Kohlenstoffatomen, wie beispielsweise Bernsteinsäure oder Adipinsäure einzusetzen, wobei die molaren Einsatzverhältnisse wiederum 10 : 90 bis 90 : 10, vorzugsweise 25 : 75 bis 75 : 25 und insbesondere 40 : 60 bis 60 : 40 betragen können. Auch ternäre Mischungen von Zimtsäure, Fettsäure und Dicarbonsäure, insbesondere Zimtsäure, gehärteter Palmfettsäure und Adipinsäure im molaren Verhältnis 1 : (0,5 bis 1,5) : (0,5 : 2,0) sind möglich. Vorzugsweise werden Mischungen von Zimtsäuren mit technischen C_{12/18}-Kokosfettsäuren und insbesondere teilgehärteter C_{16/18}-Talg- bzw. Palmfettsäuren sowie elaidinsäurereichen C_{16/18}-Fettsäurenschnitten eingesetzt. Zur Herstellung der quaternierten Ester können die Zimtsäuren bzw. die Säuregemische und das Triethanolamin im molaren Verhältnis von 1,1 : 1 bis 3 : 1 eingesetzt werden. Im Hinblick auf die anwendungstechnischen Ei-genschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mi-schungen von Mono-, Di- und Triestern mit einem durchschnittlichen Veresterungsgrad von 1,5 bis 1,9 dar. Aus anwendungstechnischer Sicht haben sich quaternierte Zimtsäuretriethanolaminestersalze der Formel (I) als besonders vorteilhaft erwiesen, in der R¹CO für einen Zimtsäurerest, R² für R¹CO, R³ für Wasserstoff, R⁴ für eine Methylgruppe, m, n und p für 0 und X für Methylsulfat steht.

Neben den quaternierten Zimtsäuretriethanolaminestersalzen kommen als Esterquats ferner auch quatemierte Estersalze von Zimtsäuren mit Diethanolalkylaminen der Formel (II) in Betracht, 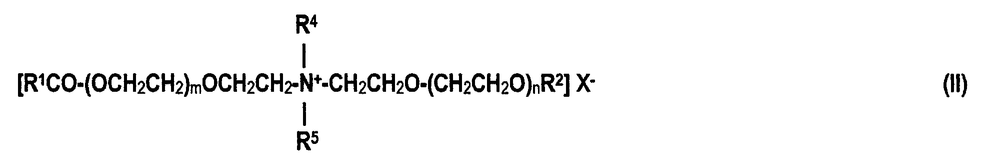 in der R¹CO für einen Rest der Zimtsäure, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.
Als weitere Gruppe geeigneter Esterquats sind schließlich die quaternierten Estersalze von Zimtsäuren mit 1,2-Dihydroxypropyldialkylaminen der Formel (III) zu nennen, 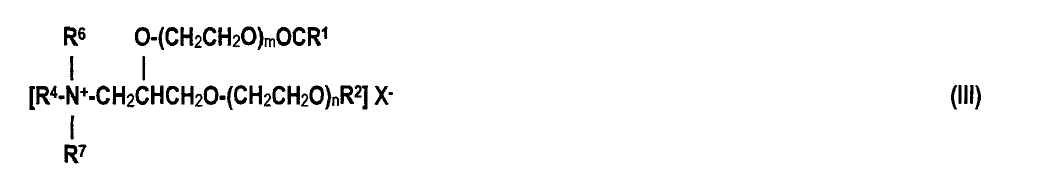 in der R¹CO für einen Rest der Zimtsäure, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich des optimalen Veresterungsgrades gelten die für (I) genannten Beispiele auch für die Esterquats der Formeln (II) und (III). Üblicherweise gelangen die Esterquats in Form 50 bis 90 Gew.-%iger alkoholischer Lösungen in den Handel, die bei Bedarf problemlos mit Wasser verdünnt werden können.

### Herstellverfahren

Ein weiterer Gegenstand der Erfindung betrifft ein Verfahren zur Herstellung der genannten Esterquats, bei dem man Zimtsäureester mit Alkanolaminen, ausgewählt aus der Gruppe, die gebildet wird von Triethanolamin, Diethanolalkylaminen und 1,2-Dihydroxypropyldialkylaminen sowie deren Gemischen, in Gegenwart von organischen Lösemitteln mit Dialkylsulfaten, vorzugsweise Dimethylsulfat, quaterniert. Bei den eingesetzten Estem kann sich die Säurekomponente - wie schon eingangs erläutert - ganz oder aber auch nur teilweise von der Zimtsäure ableiten, d.h. das Verfahren umschließt auch solche Ausführungsformen, in denen Ester von Gemischen aus (a) Zimtsäure und (b) Fettsäuren mit 6 bis 22 Kohlenstoffatomen und/oder Dicarbonsäuren mit 2 bis 12 Kohlenstoffatomen im Molverhältnis 10 : 90 bis 90 : 10 mit Alkanolaminen quaterniert werden. Als bevorzugte Alkanolamine sind Triethanolamin und Methyldiethanolamin sowie deren Gemische im Molverhältnis 10 : 90 bis 90 : 10 und vorzugsweise 40 : 60 bis 60 : 40 zu nennen. Als Lösemittel kommen neben dem bekannten Isopropylalkohol auch andere niedere Alkohole und Polyole, wie beispielsweise Ethanol, Ethylenglycol, Propylenglycol oder Glycerin in Frage. Zur Herstellung fester, schuppbarer Produkte empfiehlt es sich, die Quaternierung in Gegenwart eines unter Reaktionsbedingungen flüssigen, bei Raumtemperatur aber festen kosmetisch akzeptablen Hilfsstoffes durchzuführen. Hierzu eignen sich insbesondere Fettalkohole wie Cetylstearylalkohol oder wasserfreie Tenside wie Alkyloligoglucoside. Flüssige Mischprodukte werden erhalten, wenn man als kosmetisch akzeptable Lösemittel in der Quaternierung Partialglyceride oder kosmetische Öle, wie beispielsweise flüssige Triglyceride, Dialkylether und dergleichen verwendet. Die Veresterung und Quaternierung kann dabei in an sich bekannter Weise durchgeführt werden, wie dies beispielsweise ausführlich in den Druckschriften **DE 4308794 C1** und **DE 4335782 C1** (Henkel) beschrieben wird.

### Gewerbliche Anwendbarkeit

Die neuen Esterquats besitzen oberflächenaktive, avivierende und antistatische Eigenschaften und zudem ein hohes Absorptionsvermögen für UV-Strahlen. Zwei weitere Gegenstände der Erfindung betreffen daher ihre Verwendung als UV-Lichtschutzfilter zur Herstellung von kosmetischen und/oder pharmazeutischen Zubereitungen, speziell Sonnenschutzmitteln, sowie zur Ausrüstung von gefärbten textilen Flächengebilden, wie beispielsweise Fäden, Gamen, Geweben und Textilien, speziell Blue-Denimstoffen (Jeans) gegen Ausbleichen.

### Kosmetische und/oder pharmazeutische Zubereitungen

Die neuen Esterquats auf Basis Zimtsäure eignen sich zur Herstellung von kosmetischen und/oder pharmazeutischen Zubereitungen, wie beispielsweise Haarshampoos, Haarlotionen, Hautpflegemittel und speziell Sonnenschutzmittel in Form von Cremes und Lotionen, und können ferner als weitere Hilfs- und Zusatzstoffe milde Tenside, Ölkörper, Emulgatoren, Überfettungsmittel, Perlglanzwachse, Stabilisatoren, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, weitere UV-Lichtschutzfilter, Insektenrepellentien, Selbstbräuner, Parfümöle, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethyl-cellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat bzw. -ricinoleat eingesetzt werden. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B.

Wärme wieder abzugeben. UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäureisopentylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion;

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C). Weitere geeignete UV-Lichtschutzfilter sind der Übersicht von P.Finkel in **SÖFW-Journal 122, 543 (1996)** zu entnehmen.

Zur Verbesserung des Fließverhaltens können ferner **Hydrotrope,** wie beispielsweise Ethanol, lsopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der** Farbstoff**kommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionsternperatur-Methode.

### Beispiele

### Herstellbeispiele

**Beispiel 1.** In einem 1,5-l-Dreihalskolben mit Rührer, Tropftrichter und Destillationsaufsatz wurden 559 g (3,8 Mol) Zimtsäure und 1,2 g Hypophosphorsäure (50 Gew.-%ig) vorgelegt und auf 70°C erhitzt. Bei einem verminderten Druck von 30 mbar wurden portionsweise 279 g (1,9 Mol) Triethanolamin zugetropft und dabei die Temperatur bis auf 160°C gesteigert. Nach dem Ende der Zugabe wurde die Reaktionsmischung noch weitere 2 h bei 2 mbar gerührt, bis kein weiteres Reaktionswasser abgeschieden wurde und die Säurezahl einen Wert unterhalb von 5 mg KOH/g erreicht hatte. Anschließend wurden 670 g (0,86 Mol) des hergestellten Esters in einen zweiten Dreihalskolben überführt und bei 60°C in 120 g Isopropylalkohol gelöst. Anschließend wurden portionsweise 104 g (0,83 Mol) Dimethylsulfat zugetropft und die Mischung 4 h bei 65°C gerührt. Es wurde eine gelblich gefärbte Paste mit einem Feststoffgehalt von 90 Gew.-% erhalten.

**Beispiel 2.** Beispiel 1 wurde unter Einsatz von 865 g (3,2 Mol) teilgehärteter Talgfettsäure, 53 g (0,36 Mol) Zimtsäure, 1,5 g Hypophosphorsäure und 279 g (1,87 Mol) Triethanolamin wiederholt. 1092 g (1,82 Mol) des resultierenden Mischesters wurden anschließend in 146 g Isopropylalkohol gelöst und mit 218 g (1,73 Mol) Dimethylsulfat quaterniert. Es wurde eine gelblich gefärbte Paste mit einem Feststoffgehalt von 90 Gew.-% und einem Aktivsubstanzgehalt von 1,086 meq/g erhalten.

**Beispiel 4.** Beispiel 2 wurde unter Einsatz von 270 g (1 Mol) teilgehärteter Talgfettsäure, 148 g (1 Mol) Zimtsäure, 0,6 g Hypophosphorsäure und 157 g (1,05 Mol) Triethanolamin wiederholt. 531 g (1,05 Mol) des resultierenden Mischesters wurden anschließend in 75 g Ethanol gelöst und mit 125 g (0,99 Mol) Dimethylsulfat quaterniert. Es wurde eine gelblich gefärbte Lösung mit einem Feststoffgehalt von 90 Gew.-% und einem Aktivsubstanzgehalt von 1,1151 meq/g erhalten.

**Beispiel 5.** Beispiel 2 wurde unter Einsatz von 270 g (1 Mol) teilgehärteter Talgfettsäure, 148 g (1 Mol) Zimtsäure, 0,6 g Hypophosphorsäure und 157 g (1,05 Mol) Triethanolamin wiederholt. 420 g (1,05 Mol) des resultierenden Mischesters wurden anschließend in 75 g Cetylstearylalkohol gelöst und mit 125 g (0,99 Mol) Dimethylsulfat quaterniert. Es wurde ein gelblich gefärbtes wachsartiges Produkt mit einem Feststoffgehalt von 90 Gew.-% und einem Aktivsubstanzgehalt von 1,1151 meq/g erhalten, das sich leicht schuppen ließ.

**Beispiel 6.** Analog Beispiel 1 wurden bei 80°C 133 g (0,49 mol) teilgehärteter Palmfettsäure, 73 g (0,49 mol) Zimtsäure und 103 g (0,71 mol) Adipinsäure in Gegenwart von 1 g hypophosphoriger Säure zur Reaktion gebracht. Bei einem verminderten Druck von 35 mm Hg wurden portionsweise 210 g (1,41 mol) Triethanolamin zugegeben, die Temperatur auf 170°C gesteigert und der Druck auf 2 mm Hg abgesenkt. Nachdem die Säurezahl auf einen Wert unter 2 gefallen war, wurde das Vakuum mit Stickstoff gebrochen. 445 g (1,32) des auf diese Weise gewonnenen Esters wurden bei 60°C in 106 g Isopropylalkohol gelöst. Anschließend wurden portionsweise 158 g (1,25 mol) Dimethylsulfat hinzugegeben und die Mischung 3 h bei 65 bis 70°C gerührt. Es resultierte eine leicht gelblich gefärbte, klare Flüssigkeit mit einem Feststoffgehalt von 85 Gew.-% und einem Aktivanteil von 1,17 meq/g.

**Beispiel 7.** Analog Beispiel 6 wurden 209 g (1,4 mol) Triethanolamin, 145 g (0,98 mol) Zimtsäure und 102 g (0,7 mol) Adipinsäure umgesetzt. 370 g (1,24 mol) des resultierenden Esters wurden 91 g Isopropylalkohol gelöst und mit 148 g (1,17 mol) Dimethylsulfat quaterniert. Es resultierte eine leicht gelblich gefärbte, klare Flüssigkeit mit einem Feststoffgehalt von 87,5 Gew.-% und einem Aktivanteil von 0,9 meq/g.

**Beispiele 8 bis 11, Vergleichsbeispiel V1.** Blue Denim-Gewebe wurde im Foulard-Verfahren mit 5 Gew.-%igen Kationtensidlösungen behandelt und ausgerüstet. Nach der Zwangsapplikation wurden die Gewebe von einem Panel bestehend 6 geschulten Personen hinsichtlich ihres Weichgriffs beurteilt. Dabei bedeutet (1) = sehr weich und (4) = hart. Anschließend wurden die Testgewebe 4 Wochen mit einer 1000-W-Lampe bestrahlt, deren spektrale Verteilung der des natürlichen Sonnenlichtes entsprach. Danach wurden die Gewebe von der gleichen Testgruppe bezüglich ihrer Ausbleichung beurteilt. Dabei bedeutet (1) = keine Ausbleichung und (4) = starke Ausbleichung. Alle Angaben in Tabelle 1 stellen Mittelwerte der Testgruppe basierend auf 5 Versuchen dar. Die in den erfindungsge-mäßen Beispielen 8 bis 11 eingesetzten Esterquats entsprechen den Zimtsäurederivaten nach den Beispielen 1 bis 4; für das Vergleichsbeispiel V1 wurde ein handelsübliches Esterquat auf Basis reiner teilgehärteter Talgfettsäure eingesetzt (Dehyquart® AU 56, Pulcra S.A./Barcelona).

**Tabelle 1**

| **Weichgriff und Ausbleichung** | | | | |
|---|---|---|---|---|
| **Beispiel** | **8** | **9** | **11** | **V1** |
| Weichgriff | 1,7 | 1,4 | 1,5 | 1,4 |
| Ausbleichung | 1,1 | 1,5 | 1,3 | 3,5 |

### Rezepturbeispiele

| **Haarspülung (I)** | |
|---|---|
| Hexadecyl Polyglucose (and) Hexadecyl Alcohol | 4,0 |
| Hydrolyzed Keratin | 2,3 |
| Coco Glucosides | 2,0 |
| Zimtsäureesterquat gemäß Beispeil 1 | 1,0 |
| Polyglyceryl-3-isostearat | 1,0 |
| Decyl Oleate | 1,0 |
| Glyceryl Stearate | 0,5 |

| **Haarspülung (II)** | |
|---|---|
| Cetearyl Alcohol | 2,5 |
| Zimtsäureesterquat gemäß Beispiel 2 | 1,0 |
| Dicaprylyl Ether | 1,0 |
| Ceteareth-20 | 0,8 |
| Glyceryl Stearate | 0,5 |

| **Haarspülung (IV)** | |
|---|---|
| Cetearyl Alcohol | 2,5 |
| Hydrolyzed Collagen | 2,0 |
| Zimtsäureesterquat gemäß Beispiel 5 | 1,0 |
| Polyglyceryl-3 Diisostearat | 1,0 |
| Decyl Oleate | 1,0 |
| Ceteareth-20 | 0,8 |
| Glyceryl Stearate | 0,5 |

| **Leave-on Hair Rinse** | |
|---|---|
| Polyacrylate (and) Laureth-2 (and) Paraffinöl | 3,0 |
| Hydrolyzed Collagen | 2,0 |
| Zimtsäureesterquat gemäß Beispiel 1 | 0,8 |
| Coco Glucosides | 0,5 |
| Oleyl Erucate | 0,5 |
| Tocopherol Acetate | 0,2 |
| Ethanol | 10,0 |
| Glycerin (86 %ig) | 5,0 |

| **Haarkur (I)** | |
|---|---|
| Cetearyl Alcohol | 3,0 |
| Soja Sterol | 1,0 |
| Zimtsäureesterquat gemäß Beispiel 2 | 1,0 |
| Ceteareth-20 | 0,8 |
| Glyceryl Stearate | 0,5 |

| **Haarkur (II)** | |
|---|---|
| Cetearyl Alcohol | 2,5 |
| Zimtsäureesterquat gemäß Beispiel 5 | 1,5 |
| Ceteareth-20 | 1,0 |
| Soja Sterol | 1,0 |
| Octyldodecanol | 1,0 |
| Glyceryl Sterate | 1,0 |

| **Shampoo (I)** | |
|---|---|
| Sodium Laureth Sulfate | 25,0 |
| Coco Glucosides | 5,0 |
| Cocamidopropyl Betaine | 8,0 |
| Zimtsäureesterquat gemäß Beispiel 4 | 3,0 |
| Laureth-2 (NRE) | 1,5 |
| PPG-2-Ceteareth-9 | 1,0 |
| Parfümöl | 5,0 |

| **Shampoo (II)** | |
|---|---|
| Sodium Laureth Sulfate | 11,0 |
| Disodium Laureth Sulfosuccinate | 7,0 |
| Coco Glucosides | 4,0 |
| Zimtsäureesterquat gemäß Beispiel 1 | 1,0 |
| Hydrolyzed Collagen | 2,0 |
| NaCl | 1,6 |

| **Shampoo (III)** | |
|---|---|
| Coco Glucosides (and) Sodium Laureth Sulfate | 16,0 |
| Zimtsäureesterquat gemäß Bespiel 2 | 2,0 |
| NaCl | 2,0 |

| **Shampoo (V)** | |
|---|---|
| Sodium Laureth Sulfate | 11,0 |
| Coco Glucosides | 6,0 |
| Hydrolyzed Collagen | 2,0 |
| Zimtsäureesterquat gemäß Beispiel 4 | 2,0 |
| Triethyleneglycol Distearate (and) Sodium Laureth Sulfate | 3,0 |
| NaCl | 3,0 |

| **Shampoo (VI)** | |
|---|---|
| Ammonium Laureth Sulfate | 23,0 |
| Coco Glucosides | 4,0 |
| Cocamidopropyl Betaine | 7,0 |
| Zimtsäureesterquat gemäß Beispiel 1 | 2,0 |
| Hydrogenated Tallow Glycerides (and) Potassium Cocoyl Hydrolyzed Collagen | 5,0 |
| Glyceryl Laurate | 1,0 |
| NaCl | 3,0 |

| **Softcreme** | |
|---|---|
| Glyceryl Stearate (and) Ceteareth-12/20 (and) Cetearyl Alcohol (and) Cetyl Palmitate | 5,0 |
| Decyl Oleate | 3,0 |
| Celearyl Isononanoate | 3,0 |
| Glycerin (86 Gew.-%ig) | 3,0 |
| Zimtsäureesterquat gemäß Beispeil 2 | 40,0 |

| **Nachtcreme** | |
|---|---|
| Polyglyceryl-3 Diisostearate | 4,0 |
| Glyceryl Oleate | 2,0 |
| Bienenwachs | 7,0 |
| Dicaprylyl Ether | 5,0 |
| Octyldodecanol | 10,0 |
| Coco Caprylate Caprate | 5,0 |
| Glycerin (86 Gew.-%ig) | 5,0 |
| Magnesiumsulfat | 1,0 |
| Zimtsäureesterquat gemäß Bespiel 4 | 5,0 |

| **Sonnenschutzcreme** | |
|---|---|
| Cetearylglucoside (and) Cetearyl Alkohol | 4 |
| Hydrogenated Palm Glycerides | 2 |
| Di-n-octylcarbonat | 8 |
| Coco Glycerides | 6 |
| Octyl Methoxycinnamate | 5 |
| 4-Methylbenzyliden Camphor | 3 |
| Zimtsäureesterquat gemäß Beispiel 1 | 3 |
| Benzophenon-3 | 2 |
| Titandioxid | 1 |
| Zinkoxid | 1 |
| Octyl Triazone | 1 |
| Glycerin (86 Gew.-%ig) | 5 |

| **Sonnenschutzcreme** | |
|---|---|
| Lauryl Glucoside (and) Polyglyceryl-2-Dipolyhydroxystearate | 4 |
| Hydrogenated Palm Glycerides | 2 |
| Dicaprylyl Ether | 10 |
| Coco Glycerides | 8 |
| Octyl Methoxycinnamate | 4 |
| 4-Methylbenzyliden Camphor | 3 |
| Zimtsäureesterquat gemäß Beispiel 2 | 3 |
| Benzophenon-3 | 2 |
| Titandioxid | 1 |
| Zinkoxid | 1 |
| Octyl Triazone | 1 |
| Glycerin (86 Gew.-%ig) | 5 |

## Patentansprüche

1. Esterquats, deren Säurekomponente sich ganz oder teilweise von Zimtsäuren ableitet, **dadurch gekennzeichnet, daß** sie der Formel **(I)** folgen, 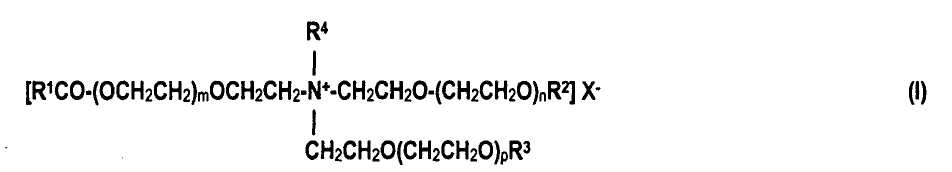 in der R¹CO für einen Rest der Zimtsäure, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

2. Quaternierte Estersalze, deren Säurekomponente sich ganz oder teilweise von Zimtsäuren ableitet, **dadurch gekennzeichnet, daß** sie der Formel **(II)** folgen, 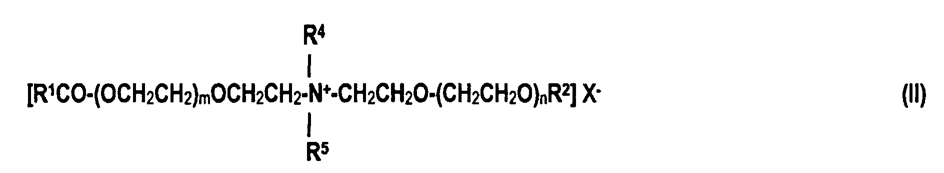 in der R¹CO für einen Rest der Zimtsäure, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

3. Quaternierte Estersalze, deren Säurekomponente sich ganz oder teilweise von Zimtsäuren ableitet, **dadurch gekennzeichnet, daß** sie der Formel (III) folgen, 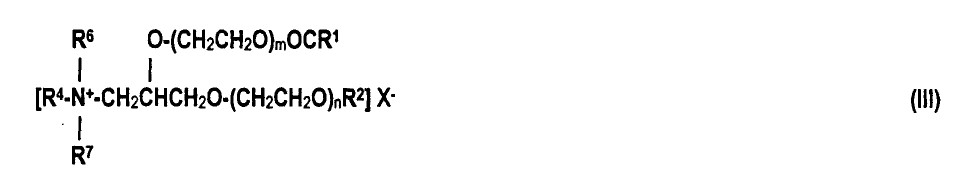 in der R¹CO für einen Rest der Zimtsäure, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Verfahren zur Herstellung von Esterquats und/oder quaternierten Estersalzen, bei dem man Ester der Zimtsäure mit Alkanolaminen, ausgewählt aus der Gruppe, die gebildet wird von Triethanolamin, Diethanolalkylaminen und 1,2-Dihydroxypropyldialkylaminen sowie deren Gemischen, in Gegenwart von organischen Lösemitteln mit Dialkylsulfaten quaterniert.

5. Verfahren nach Anspruch 4, **dadurch gekennzeichnet, daß** man Ester einsetzt, deren Säurekomponenten sich von Mischungen von (a) Zimtsäure mit (b) Fettsäuren mit 6 bis 22 Kohlenstoffatomen und/oder Dicarbonsäuren mit 2 bis 12 Kohlenstoffatomen im molaren Verhältnis 10 : 90 bis 90 : 10 ableiten.

6. Verfahren nach den Ansprüchen 4 und 5, **dadurch gekennzeichnet, daß** man die Quaternierung in Gegenwart von Ethanol, Isopropylalkohol, Ethylenglycol, Propylenglycol, Glycerin oder deren Gemischen durchführt.

7. Verfahren nach den Ansprüchen 4 bis 5, **dadurch gekennzeichnet, daß** man die Quaternierung in Gegenwart von Fettalkoholen, Alkyloligoglucosiden, Partialglyceriden und/oder kosmetischen Ölen durchführt.

8. Verwendung von Esterquats und/oder quaternierten Estersalzen nach den Ansprüchen 1 bis 3 als UV-Lichtschutzfilter zur Herstellung von kosmetischen und/oder pharmazeutischen Zubereitungen.

9. Verwendung von Esterquats und/oder quaternierten Estersalzen nach den Ansprüchen 1 bis 3 zur Ausrüstung von gefärbten textilen Flächengebilden gegen Ausbleichen.

## Claims

1. Esterquats of which the acid component is completely or partly derived from cinnamic acids, **characterized in that** they correspond to formula **(I)**: 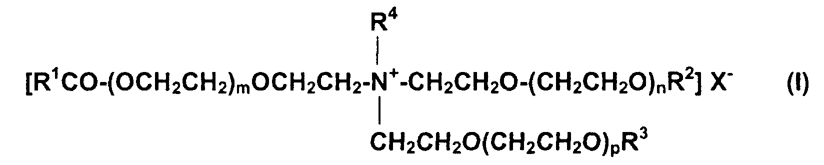 in which R¹CO is a residue of cinnamic acid, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate.

2. Quaternized ester salts of which the acid component is completely or partly derived from cinnamic acids, **characterized in that** they correspond to formula **(II)**: 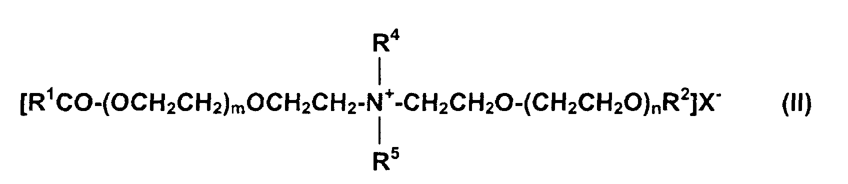 in which R¹CO is a residue of cinnamic acid, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate.

3. Quaternized ester salts of which the acid component is completely or partly derived from cinnamic acids, **characterized in that** they correspond to formula **(III)**: 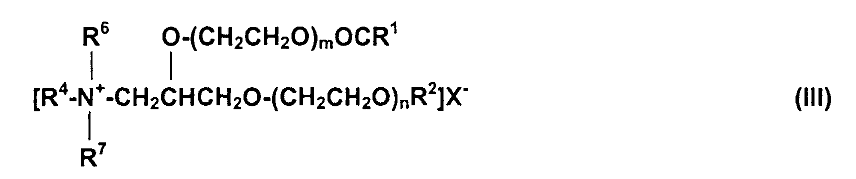 in which R¹CO is a residue of cinnamic acid, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate.

4. A process for the production of esterquats and/or quaternized ester salts, in which esters of cinnamic acid with alkanolamines selected from the group consisting of triethanolamine, diethanol alkylamines and 1,2-dihydroxypropyl dialkylamines and mixtures thereof are quaternized with dialkyl sulfates in the presence of organic solvents.

5. A process as claimed in claim 4, **characterized in that** esters of which the acid components are derived from mixtures of (a) cinnamic acid with (b) fatty acids containing 6 to 22 carbon atoms and/or dicarboxylic acids containing 2 to 12 carbon atoms in a molar ratio of 10:90 to 90:10.

6. A process as claimed in claims 4 and 5, **characterized in that** the quaternization is carried out in the presence of ethanol, isopropyl alcohol, ethylene glycol, propylene glycol, gycerol or mixtures thereof.

7. A process as claimed in claims 4 to 5, **characterized in that** the quaternization is carried out in the presence of fatty alcohols, alkyl oligoglucosides, partial glycerides and/or cosmetic oils.

8. The use of the esterquats and/or quaternized ester salts claimed in claims 1 to 3 as UV filters for the production of cosmetic and/or pharmaceutical preparations.

9. The use of the esterquats and/or quaternized ester salts claimed in claims 1 to 3 for treating dyed textile materials against fading.

## Revendications

1. Esters quaternaires, dont le composant d'acide est dérivé entièrement ou partiellement d'acides cinnamiques,
**caractérisés en ce qu'**ils répondent à
1a formule (I) 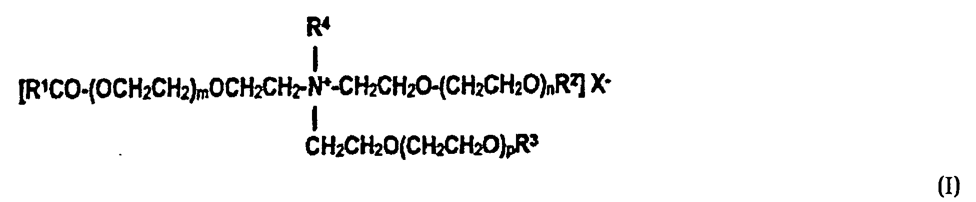 dans laquelle R¹CO représente un reste d'acide cinnamique, R² et R³ représentent, indépendamment l'un de l'autre, l'hydrogène ou R¹CO, R⁴ représente un reste alkyle comportant de 1 à 4 atomes de carbone ou un groupe (CH₂CH₂O)qH ; m, n et p ont pour somme 0 ou un nombre allant de 1 à 12, q représente un nombre allant de 1 à 12, et X représente un halogénure, sulfate d'alkyle ou phosphate d'alkyle.

2. Sels d'esters quaternisés dont le composant acide est entièrement ou partiellement dérivé d'acides cinnamiques,
**caractérisés en ce qu'**ils répondent à
la formule **(II)** 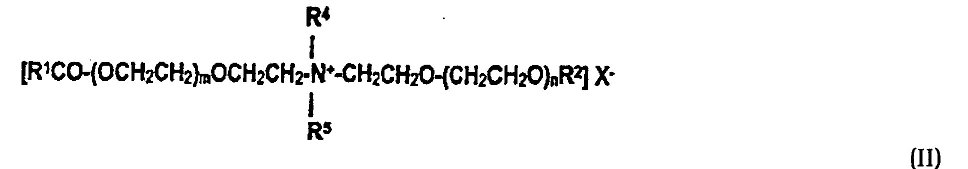 dans laquelle R¹CO représente un reste d'acide cinnamique, R² représente l'hydrogène ou R¹CO, R4 et R5 représentent, indépendamment l'un de l'autre, des restes alkyle comportant de 1 à 4 atomes de carbone, m et n ont pour somme 0 ou un nombre allant de 1 à 12, et X représente un halogénure, sulfate d'alkyle ou phosphate d'alkyle.

3. Sels d'esters quaternisés dont le composant acide est entièrement ou partiellement dérivé d'acides cinnamiques,
**caractérisés en ce qu'**ils répondent à
la formule (II) 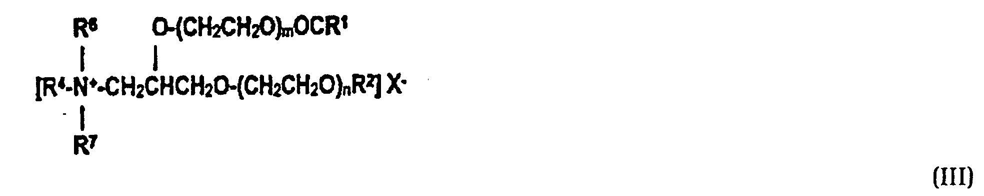 dans laquelle R¹CO représente un reste d'acide cinnamique, R2 représente l'hydrogène ou R¹CO; R4, R6 et R7 représentent, indépendamment les uns des autres, des restes alkyle comportant de 1 à 4 atomes de carbone, m et n ont pour somme 0 ou un nombre allant de 1 à 12, et X représente un halogénure, sulfate d'alkyle ou phosphate d'alkyle.

4. Procédé de production d'esters quaternaires et/ou de sels d'esters quaternisés,
selon lequel on quaternise avec des sulfates de dialkyle des esters de l'acide cinnamique avec des alcanolamines, sélectionnées dans le groupe formé de la triéthanolamine, des diéthanolalkylamines et des 1,2-dihydroxypropyldialkylamines, ainsi que leurs mélanges, en présence de solvants organiques.

5. Procédé selon la revendication 4,
**caractérisé en ce qu'**
on utilise des esters dont les composants acides sont dérivés de mélanges (a) d'acide cinnamique avec (b) des acides gras comportant de 6 à 22 atomes de carbone et/ou des acides dicarboniques comportant de 2 à 12 atomes de carbone, dans un rapport molaire allant de 10 : 90 à 90 : 10.

6. Procédé selon les revendications 4 et 5,
**caractérisé en ce qu'**
on effectue la quaternisation en présence d'éthanol, d'isopropylalcool, d'éthylèneglycol, de propylèneglycol, de glycérine ou de leurs mélanges.

7. Procédé selon les revendications 4 et 5,
**caractérisé en ce qu'**
on effectue la quaternisation en présence d'alcools gras, d'oligoglucosides d'alkyle, de glycérides partiels et/ou d'huiles cosmétiques.

8. Utilisation d'esters quaternaires, et/ou de sels d'esters quaternisés selon les revendications 1 à 3, comme filtres de protection contre la lumière ultraviolette en vue de la production de préparations cosmétiques et/ou pharmaceutiques.

9. Utilisation d'esters quaternaires, et/ou de sels d'esters quaternisés selon les revendications 1 à 3, comme apprêt de protection de structures superficielles textiles teintes contre la décoloration.
